# EUROPEAN PATENT APPLICATION

(11) **EP 3 677 255 A1**
(43) Date of publication of application: **08.07.2020**
(21) Application number: 20150056.8
(22) Date of filing: 02.01.2020
(51) Int. Cl.: A61K 31/015, A61K 31/12, A61K 31/165, A61K 31/426, A61K 31/5375, A61P 35/00

(54) **METHOD FOR TREATING A BRAIN CANCER WITH A SUBSTITUTED PHENYL-PROPENAL MOIETY**

(30) Priority: 04.01.2019 US 201962788570 P
(71) Applicant: Allianz Pharmascience Ltd, 10682 Taipei (TW)
(72) Inventor: CHAN, Hardy W., Redwood City CA 94065 (US); CHEN, Tzu Chi, 116 Taipei (TW)
(74) Representative: Fuchs Patentanwälte Partnerschaft mbB

(57) **Abstract**

The invention provides a method for treating a brain cancer in a subject in need of such treatment, comprising administrating to said subject a compound with (substituted phenyl)-propenal moiety.

## Description

### FIELD OF THE INVENTION

This invention relates to a treating method, more specifically, the present invention provides a method for treating a brain cancer with compounds with at least one (substituted phenyl)-propenal moiety and use thereof. This invention further relates to a pharmaceutical composition comprising the compounds for use in a method for treating a brain cancer in a subject in need of such treatment.

### BACKGROUND OF THE INVENTION

It is well known that certain natural products may possess therapeutic effects, which has lead to their use in the treatment and prevention of human diseases across many cultures (e.g., Chinese herbal medicines and many other folk medicines). The effectiveness of such treatments has lead the pharmaceutical industry to seek and isolate active compounds from these natural products and develop the active ingredients as therapeutic or prophylactic drugs for the treatment and prevention of a variety of diseases or medical conditions. Thus, many commonly used pharmaceuticals have been developed or have arisen from natural products. However, compounds isolated from natural products are known to play certain physiological function(s) in its native host; whereas their therapeutic effects against human diseases are not readily apparent. Historically, such therapeutic treatments were derived merely by accumulated experiences or "trial and error" in humans. Moreover, because such compounds were not initially created for use in humans, the compounds in their native form are frequently not in the most optimal form, both in structure as well as efficacy, to treat human diseases. However, today's modern chemistry technology, including analytical and synthetic chemistries, together with the advances in medicinal biology have made it possible for one to dissect a chemical structure and localize a "pharmacophore" (a core structure that is essential for the therapeutic activity) within a compound such as one isolated from a natural product; furthermore, these new techniques allow one to synthesize new compounds, based on the structure of a pharmacophore, that possess optimal or even better therapeutic efficacy.

Compound curcumin (existing as a major pigment in a turmeric plant) and many of its analogs have been reported to possess numerous biological activities *in vitro,* such as, anti-oxidant, anti-inflammatory, anti-tumor, and antiangiogenesis activities; but neither curcumin nor its analogues have been developed into a therapeutic drug to treat human diseases. This indicates curcumin in its native form is probably not an optimal molecule for development into a therapeutic drug. Therefore, developing suitable curcumin analogues for clinical uses is needed.

Glioblastoma multiforme (GBM) is a lethal disease, and patients survive an average of 14 months. Most patients do not survive beyond two years (Agnihotri S, Burrell KE, Wolf A, et al. Glioblastoma, a brief review of history, molecular genetics, animal models and novel therapeutic strategies. Arch Immunol Ther Exp (Warsz). 2013;61(1):25-41; Wilson TA, Karajannis MA, Harter DH. Glioblastoma multiforme: State of the art and future therapeutics. Surg Neurol Int. 2014;5:64).

Surgical resection alone results in a median survival of approximately 6 months (Wilson TA, Karajannis MA, Harter DH. Glioblastoma multiforme: State of the art and future therapeutics. Surg Neurol Int. 2014;5:64). It has been further demonstrated that concomitant treatment with radiation and temozolomide (TMZ) followed by adjuvant temozolomide increases median overall survival (OS) from 12.1 to 14.6 months compared to that of patients who receive radiation alone (Agnihotri S, Burrell KE, Wolf A, et al. Glioblastoma, a brief review of history, molecular genetics, animal models and novel therapeutic strategies. Arch Immunol Ther Exp (Warsz). 2013;61(1):25-41). Despite intense initial treatment, tumor recurrence is almost inevitable and the 5 year survival for GBM patients remains under 9% (Agnihotri S, Burrell KE, Wolf A, et al. Glioblastoma, a brief review of history, molecular genetics, animal models and novel therapeutic strategies. Arch Immunol Ther Exp (Warsz). 2013;61(1):25-41; Ostrom QT, Gittleman H, Xu J, et al. CBTRUS Statistical Report: Primary Brain and Other Central Nervous System Tumors Diagnosed in the United States in 2009-2013. Neuro-Oncology. 2016;18(suppl_5):v1-v75). The effectiveness of current conventional therapies remains poor, resulting in an unmet medical need for the treatment of GBM.

### SUMMARY OF THE INVENTION

The invention is to provide a method and/or a pharmaceutical composition for treating a brain cancer in a subject in need of such treatment, comprising administrating to said subject a pharmaceutical composition comprising an effective amount of a compound according to formula VIII and optionally a pharmaceutically acceptable carrier or excipient; 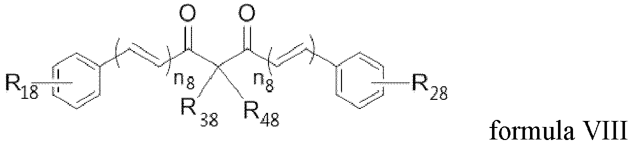
R₁₈ and R₂₈ are mono- or di-substituted groups and independently selected from the group consisting of a methoxy group, a hydroxyl group, and an alkyl sulfonyl group;
R₃₈ is selected from the group consisting of 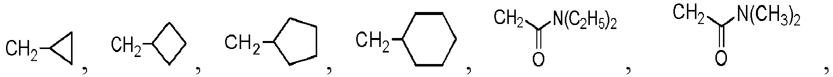 (CH₂)₃CH₃, 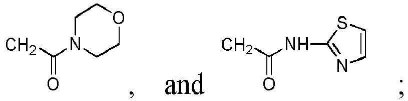 and
R₄₈ is selected from the group consisting of CH₃, H, F and Cl; and
n₈ is 1 or 2.

The present invention is described in detail in the following sections. Other characteristics, purposes and advantages of the present invention can be found in the detailed description and claims.

### BRIEF DESCRIPTION OF THE DRAWINGS

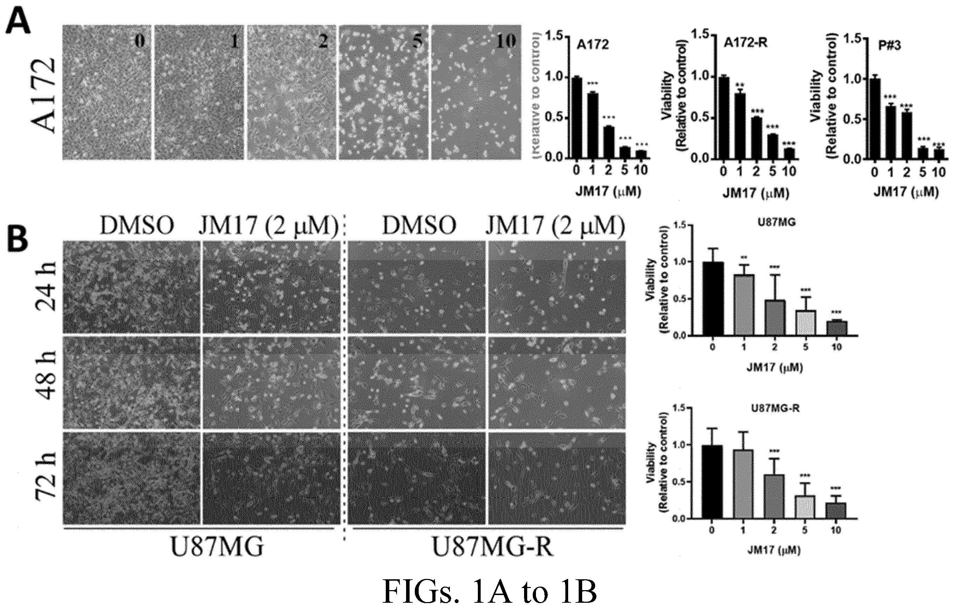

FIGs. 1A to 1B show that ASC-JM17 (JM17) inhibits proliferation of glioblastoma cells with or without temozolomide (TMZ) -resistance. FIG. 1A: After treatment for 48 h, TMZ-sensitive A172 and Pt#3, and TMZ-resistant A172-R cells were harvested for MTT assay. The difference between 2 groups was analyzed by Student's t test. FIG. 1B: Phase images of cells treated with JM17 or vehicle (DMSO) as indicated. Cells were photographed. Right panel: After treatment for 48 h, TMZ-sensitive U87MG (top), and -resistant U87MG-R (bottom) cells were harvested for MTT assay (*P<0.05, **P<0.01, ***P<0.001).

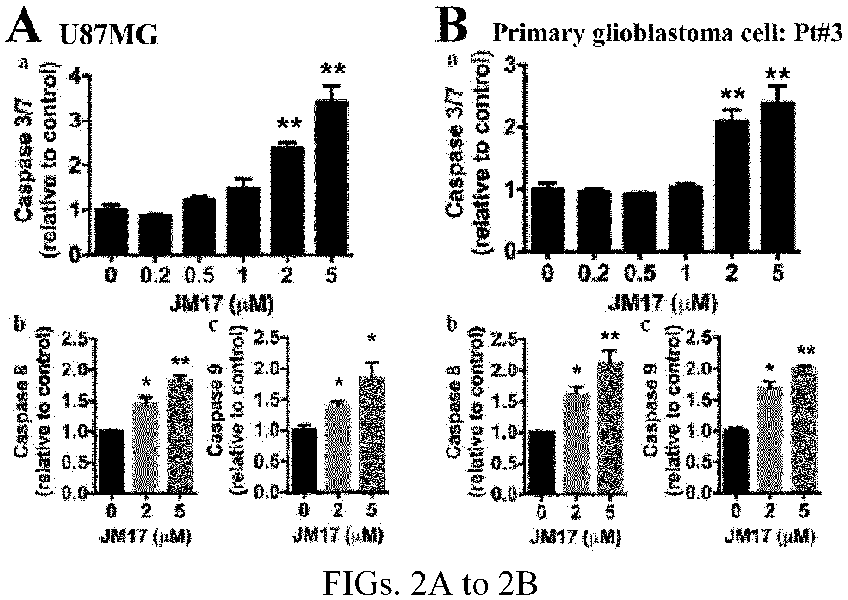

FIGs. 2A to 2B show that ASC-JM17 (JM17) induces apoptosis in glioblastoma cells as evidenced by increased activities of caspases 3/7, 8 and 9. Caspase-Glo® 3/7, 8 and 9 assay kits were used (Promega® Inc.) and the signal representing caspases was detected by the luminometer (Promega® Inc.). The activities of caspases 3/7, caspase 8 and caspase 9 were determined in the supernatants of cultured glioblastoma cells that were treated with JM17 for 48h (*P<0.05, **P<0.01).

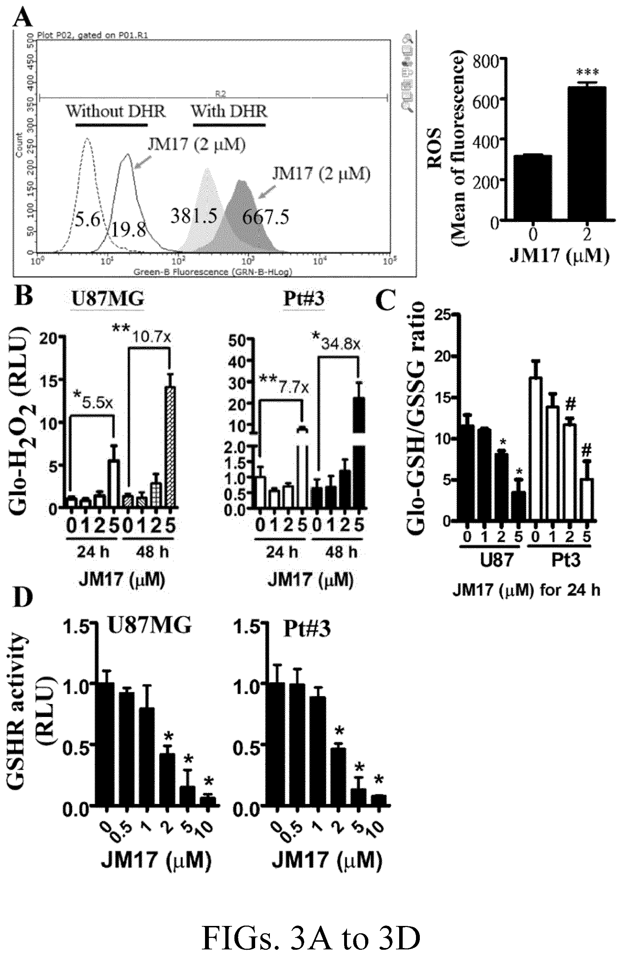

FIGs. 3A to 3D show that ASC-JM17 (JM17) increases ROS production and induces oxidative stress in the GBM cells. FIG. 3A: U87MG cells were treated for 48 hours before harvested and the ROS production was measured by flow cytometric assay using dihydrorhodamine 123 (DHR). The histogram of the representative experiment shows a fluorescence shift to the right in JM17 treated cells compared to untreated controls. The results are quantified as the mean fluorescence and presented in the right panel. Further, U87MG and primary human GBM cells Pt#3 were treated with increasing concentrations of JM17 and H₂O₂ level (FIG. 3B), GSH/GSSG ratio (FIG. 3C) and GSHR activity (FIG. 3D) were measured. *P and #P indicate statistical significance between untreated controls and the treatment for U87MG and Pt#3, respectively (*P, #P<0.05, **P<0.01).

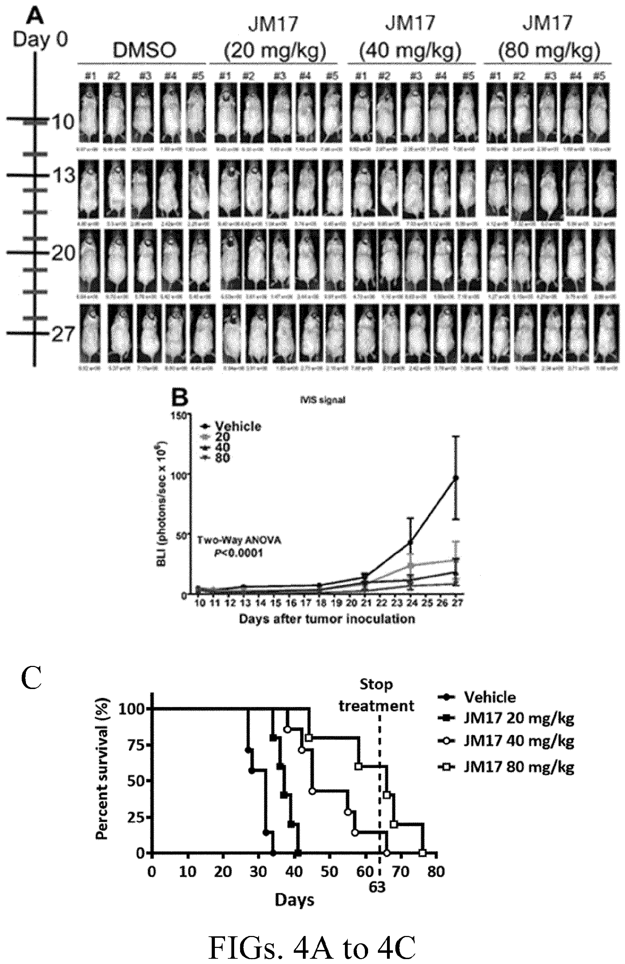

FIGs. 4A to 4C show therapeutic effect of ASC-JM17 (JM17) on the growth of glioblastoma *in vivo.* FIG. 4A: Luciferase-expressed U87MG cells (2x10⁵) were intracranially transplanted into brains of nude mice. After 10 days, luciferase activity representing tumor size was monitored by IVIS200 system on Days 10, 13, 20 and 27. Experimental mice were administrated intravenously with vehicle or JM17 at the dose of 20 mg/kg, 40 mg/kg and 80 mg/kg three times per week from Day 10. Left: The time line for IVIS200 evaluation and drug injection. FIG. 4B: The statistical analysis for luciferase activity analyzed by two-way ANOVA (***P<0.0001). FIG. 4C: The comparison of survival period was performed using Log-Rank Test (***P<0.0001). JM17 administration was terminated on Day 63.

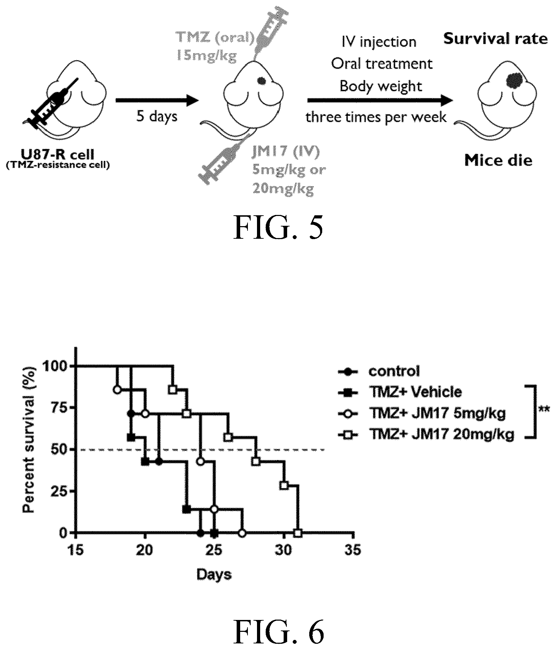

FIG. 5 shows schematic presentation of the experimental design and execution in the glioblastoma multiforme (GBM) animal model. Animal survival was evaluated from the first day of treatment until death. Body weight was measured twice a week.
FIG. 6 shows effect of ASC-JM17 (JM17) treatment on animal survival. TMZ-resistant GBM inoculated orthotopic mice were randomly grouped and treated with TMZ or TMZ plus JM17. Survival was plotted using a Kaplan-Meier curve (**, p < 0.01).

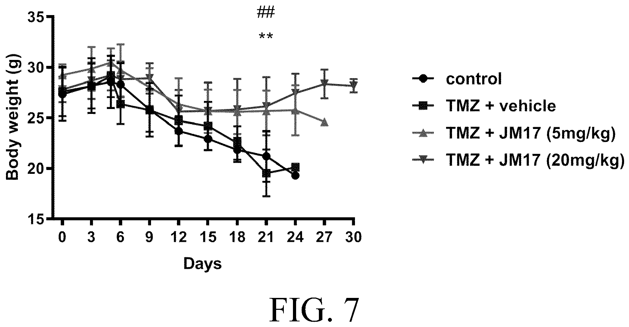

FIG. 7 shows that ASC-JM17 (JM17) prevents the loss of body weight in GBM inoculated mice. The body weight of TMZ-resistant GBM inoculated orthotopic mice treated with TMZ or TMZ plus JM17 were measured twice a week. Body weight statistics at day 21 were analyzed with student t-test. ##p <0.01 TMZ + vehicle vs TMZ + JM17 (5 mg/kg); **p<0.01 TMZ + vehicle vs TMZ + JM17 (20 mg/kg).

### DETAILED DESCRIPTION OF THE INVENTION

The present invention can be more readily understood by reference to the following detailed description of various embodiments of the invention, the examples, and the chemical drawings and tables with their relevant descriptions. It is to be understood that unless otherwise specifically indicated by the claims, the invention is not limited to specific preparation methods, carriers or formulations, or to particular modes of formulating the compound of the invention into products or compositions intended for topical, oral or parenteral administration, because as one of ordinary skill in the relevant arts is well aware, such things can, of course, vary. It is also to be understood that the terminology used herein is for the purpose of describing particular embodiments only and is not intended to be limiting.

The invention provides a method for treating a brain cancer in a subject in need of such treatment, comprising administrating to said subject a pharmaceutical composition comprising an effective amount of a compound with (substituted phenyl)-propenal moiety, preferably according to formula VIII and optionally a pharmaceutically acceptable carrier or excipient; 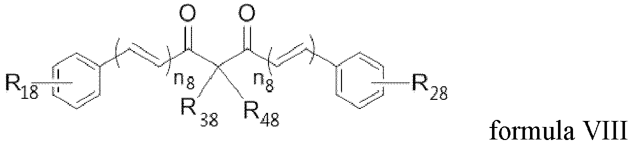
R₁₈ and R₂₈ are mono- or di-substituted groups and independently selected from the group consisting of a methoxy group, a hydroxyl group, and an alkyl sulfonyl group;
R₃₈ is selected from the group consisting of 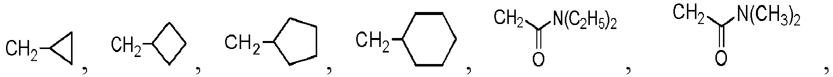 (CH₂)₃CH₃, 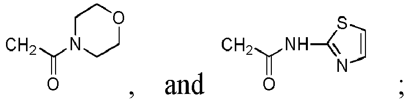 and
R₄₈ is selected from the group consisting of CH₃, H, F and Cl; and
n₈ is 1 or 2.

The term "(substituted phenyl)-propenal moiety" as used herein refers to a composition including a phenyl group having attached thereto a propenal moiety (when m equals 1) and an alkoxy or hydroxy moiety, or an alkyl or substituted alkyl moiety. The substitutions may be positioned meta or para or ortho with respect to the propenal moiety as used herein and refers to a general formula 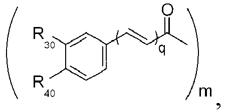 where q may be any number of 1, 2, 3 or 4; and m may be any number of 1, 2, 3, 4, or more.

The term "alkyl" as used herein refers to a straight or branched hydrocarbon chain radical consisting solely of carbon and hydrogen atoms, containing no unsaturation, having from one to ten carbon atoms, and which is attached to the rest of the molecule by a single bond, e.g. methyl, ethyl, n-propyl, 1-methylethyl (iso-propyl), n-butyl, n-pentyl, 1,1-dimethylethyl (t-butyl), and the like.

The term "alkenyl" as used herein refers to a straight or branched hydrocarbon chain radical consisting solely of carbon and hydrogen atoms, containing at least one double bond, having from two to ten carbon atoms, and which is attached to the rest of the molecule by a single bond or a double bond, e.g. ethenyl, prop-1-enyl, pent-1-enyl, penta-1,4-dienyl, and the like.

The term "alkenylene" as used herein refers to a straight or branched hydrocarbon chain which contains a carbon-to-carbon double bond and is represented by the formula CₚH₂ₚ₋₂, wherein hydrogen may be replaced by an additional carbon-to-carbon double bond or a monovalent substituent, e.g. ethenylene, prop-1-enylene and the like.

The term "alkoxy" as used herein refers to the radical having the formula -OR wherein R is an alkyl, haloalkyl or cycloalkyl. An "optionally substituted alkoxy" refers to the radical having the formula -OR' wherein R' is an optionally substituted alkyl as described herein.

The term "alkynl" as used herein refers to a straight or branched hydrocarbon chain radical consisting solely of carbon and hydrogen atoms, containing at least one triple bond, having from two to ten carbon atoms, and which is attached to the rest of the molecule by a single bond or a triple bond, e.g. ethynyl, prop-1-ynyl, but-1-ynyl, pent-1-ynyl, pent-3-ynyl and the like.

The term "aryl" as used herein refers to a radical of carbocyclic ring system wherein at least one of the rings is aromatic. The aryl may be fully aromatic or may contain an aromatic ring in combination with a non-aromatic ring. A "biaryl system" is a compound that includes at least two aryl groups.

The term "cycloalkyl" as used herein refers to a stable monovalent monocyclic or bicyclic hydrocarbon radical consisting solely of carbon and hydrogen atoms, having from three to ten carbon atoms, and which is saturated and attached to the rest of the molecule by a single bond, e.g. cyclopropyl, cyclobutyl, cyclopentyl, cyclohexyl, and the like.

The term "di-ketone bridge," or "ketone-enol bridge" as used herein, refers to a straight or branched hydrocarbon chain including two ketones or an enol positioned in close proximity to a ketone respectively. The "di-ketone bridge" or "ketone-enol bridge" is positioned between at least two aryl moieties.

The term "hydroxyalkyl" as used herein refers to a straight or branched hydroxy substituted hydrocarbon chain radical having from one to ten carbon atoms, e.g. -CH₂OH, -(CH₂)₂OH and the like.

In one preferred embodiment of the invention, the compounds including 4,4-disubstituted 1,7-bis-(3,4-dimethoxyphenyl)-hepta-1,6-diene-3,5-dione and 6,6-disubstituted 1,11-bis(substituted phenyl)-undeca-1,3,8,10-tetraene-5,7-dione scaffolds are as shown in Table 1.

**Table 1:**

| | | | | | | |
|---|---|---|---|---|---|---|
| 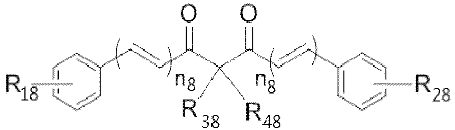 | | | | | | |

| **Compound ID** | **R₁₈** | **R₂₈** | **R₃₈** | **R₄₈** | **n₈** | **formula** |
|---|---|---|---|---|---|---|
| 1 | 3'4'-OCH₃ | 3'4'-OCH₃ | 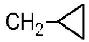 | CH₃ | 1 | C₂₈H₃₂O₆ 464.55 |
| 2 | 3'4'-OCH₃ | 3'4'-OCH₃ | 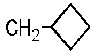 | CH₃ | 1 | C₂₉H₃₄O₆ 478.5767 |
| 3 | 3'4'-OCH₃ | 3'4'-OCH₃ | 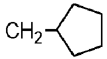 | H | 1 | C₂₉H₃₄O₆ 478.58 |
| 4 | 3'4'-OCH₃ | 3'4'-OCH₃ | 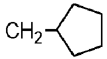 | CH₃ | 1 | C₃₀H₃₆O₆ 492.60 |
| 5 | 3'4'-OCH₃ | 3'4'-OCH₃ | 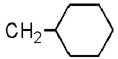 | CH₃ | 1 | C₃₁H₃₈O₆ 506.63 |
| 6 | 3'4'-OCH₃ | 3'4'-OCH₃ | 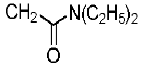 | CH₃ | 1 | C₃₀H₃₇NO₇ 523.62 |
| 7 | 3'4'-OCH₃ | 3'4'-OCH₃ | 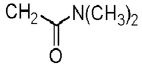 | CH₃ | 1 | C₂₈H₃₃NO₇ 495.56 |
| 8 | 3'4'-OCH₃ | 3'4'-OCH₃ | (CH₂)₃CH₃ | CH₃ | 1 | C₂₈H₃₄O₆ 466.57 |
| 9 | 3'4'-OCH₃ | 3'4'-OCH₃ | 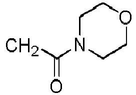 | H | 1 | C₂₉H₃₃NO₈ 523.57 |
| 10 | 3'4'-OCH₃ | 3'4'-OCH₃ | 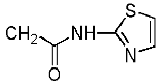 | H | 1 | C₂₈H₂₈N₂O₇S 536.60 |
| 11 | 3'4'-CH₃ | 3'4'-CH₃ | 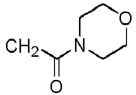 | CH₃ | 1 | C₃₀H₃₅NO₈ 537.60 |
| 12 | 3'4'-OCH₃ | 3'4'-OCH₃ | 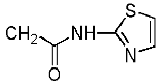 | CH₃ | 1 | C₂₉H₃₀N₂O₇S 550.62 |
| 13 | 3'4'-OCH₃ | 3'4'-OCH₃ | 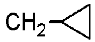 | F | 1 | C₂₇H₂₉FO₆ 468.51 |
| 14 | 3'4'-OCH₃ | 3'4'-OCH₃ | 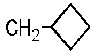 | F | 1 | C₂₈H₃₁FO₆ 482.54 |
| 15 | 3'4'-OCH₃ | 3'4'-OCH₃ | 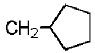 | F | 1 | C₂₉H₃₃FO₆ 496.57 |
| 16 | 3'4'-OCH₃ | 3'4'-OCH₃ | 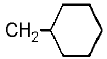 | F | 1 | C₃₀H₃₅FO₆ 510.59 |
| 17 | 3'4'-OCH₃ | 3'4'-OCH₃ | 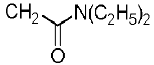 | F | 1 | C₂₉H₃₄FNO₇ 527.58 |
| 18 | 3'4'-OCH₃ | 3'4'-OCH₃ | 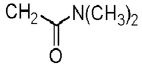 | F | 1 | C₂₇H₃₀FNO₇ 499.53 |
| 19 | 3'4'-OCH₃ | 3'4'-OCH₃ | (CH₂)₃CH₃ | F | 1 | C₂₇H₃₁FO₆ 470.53 |
| 20 | 3'4'-OCH₃ | 3'4'-OCH₃ | 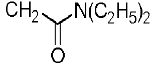 | Cl | 1 | C₂₉H₃₄ClNO₇ 544.04 |
| 21 | 3'4'-OCH₃ | 3'4'-OCH₃ | 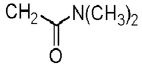 | Cl | 1 | C₂₇H₃₀ClNO₇ 515.98 |
| 22 | 3'4'-OCH₃ | 3'4'-OCH₃ | CH₂CH₂COOC₂H₅ | CH₃ | 1 | C₂₉H₃₄O₈ 510.58 |
| 23 | 3'4'-OCH₃ | 3'4'-OCH₃ | CH₂CH₂COOC₂H₅ | F | 1 | C₂₈H₃₁FO₈ 514.54 |
| 24 | 3'4'-OCH₃ | 3'4'-OCH₃ | CH₂CH₂COOH | CH₃ | 1 | C₂₇H₃₀O₈ 482.52 |
| 25 | 3'4'-OCH₃ | 3'4'-OCH₃ | CH₂CH₂COOH | F | 1 | C₂₆H₂₇FO₈ 486.49 |
| 26 | 3'OCH₃,4'OH | 3'OCH₃,4'OH | CH₂CH₂COOC₂H₅ | CH₃ | 1 | C₂₇H₃₀O₈ 482.52 |
| 27 | 3'OCH₃,4'OH | 3'OCH₃,4'OH | CH₂CH₂COOC₂H₅ | F | 1 | C₂₆H₂₇FO₈ 486.49 |
| 28 | 3'-OCH₃ | 3'-OCH₃ | 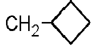 | CH₃ | 1 | C₂₇H₃₀O₄ 418.52 |
| 29 | 3'-OH | 3'-OH | 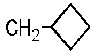 | CH₃ | 1 | C₂₅H₂₆O₄ 390.47 |
| 30 | 3'-OCH₃ | 3'-OSO₂C₂H₅ | 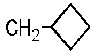 | CH₃ | 1 | C₂₈H₃₂O₆S 496.62 |
| 31 | 3'-OSO₂C₂H₅ | 3'-OSO₂C₂H₅ | 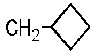 | CH₃ | 1 | C₂₉H₃₄O₈S₂ 574.71 |
| 32 | 3'4'-OCH₃ | 3'4'-OCH₃ | 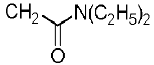 | CH₃ | 2 | C₃₄H₄₁NO₇ 575.69 |
| 33 | 3'4'-OCH₃ | 3'4'-OCH₃ | 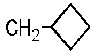 | F | 2 | C₃₂H₃₅FO₆ 534.62 |
| 34 | 3'4'-OCH₃ | 3'4'-OCH₃ | 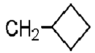 | H | 1 | C₂₈H₃₂O₆ 464.55 |

In another preferred embodiment of the invention, the compound is formula VIII, R₁₈ and R₂₈ are di-substituted methoxy groups, R₃₈ is 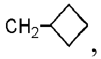 R₄₈ is H, and n₈ is 1, referred as ASC-JM17.

The invention also provides a composition comprising the compound with (substituted phenyl)-propenal moiety. The composition according to the invention is preferably a pharmaceutical composition, food composition or a cosmetic composition.

Preferably, the pharmaceutical composition comprises the compound with (substituted phenyl)-propenal moiety and optionally a pharmaceutically acceptable carrier or excipient.

Preferably, the pharmaceutical composition comprises an effective amount of the compound.

Often, ranges are expressed herein as from "about" one particular value and/or to "about" another particular value. When such a range is expressed, an embodiment includes the range from the one particular value and/or to the other particular value. Similarly, when values are expressed as approximations, by use of the word "about," it will be understood that the particular value forms another embodiment. It will be further understood that the endpoints of each of the ranges are significant both in relation to and independently of the other endpoint.

"Optional" or "optionally" means that the subsequently described event or circumstance may or may not occur, and that the description includes instances where said event or circumstance occurs and instances where it does not. For example, the phrase "optionally comprising an agent" means that the agent may or may not exist.

It must be noted that, as used in the specification and the appended claims, the singular forms "a," "an" and "the" include plural referents unless the context clearly dictates otherwise. Thus, unless otherwise required by context, singular terms shall include the plural and plural terms shall include the singular.

The term "subject" as used herein denotes any animal, preferably a mammal, and more preferably a human. The examples of subjects include humans, non-human primates, rodents, guinea pigs, rabbits, sheep, pigs, goats, cows, horses, dogs and cats.

The term "effective amount" of an active ingredient as provided herein means a sufficient amount of the ingredient to provide the desired regulation of a desired function. As will be pointed out below, the exact amount required will vary from subject to subject, depending on the disease state, physical conditions, age, sex, species and weight of the subject, the specific identity and formulation of the composition, etc. Dosage regimens may be adjusted to induce the optimum therapeutic response. For example, several divided doses may be administered daily or the dose may be proportionally reduced as indicated by the exigencies of the therapeutic situation. Thus, it is not possible to specify an exact "effective amount." However, an appropriate effective amount can be determined by one of ordinary skill in the art using only routine experimentation.

The term "treating" or "treatment" as used herein denotes reversing, alleviating, inhibiting the progress of, or improving the disorder, disease or condition to which such term applies, or one or more symptoms of such disorder, disease or condition.

The term "carrier" or "excipient" as used herein refers to any substance, not itself a therapeutic agent, used as a carrier and/or diluent and/or adjuvant, or vehicle for delivery of a therapeutic agent to a subject or added to a formulation to improve its handling or storage properties or to permit or facilitate formation of a dose unit of the composition into a discrete article such as a capsule or tablet suitable for oral administration. Suitable carriers or excipients are well known to persons of ordinary skill in the art of manufacturing pharmaceutical formulations or food products. Carriers or excipients can include, by way of illustration and not limitation, buffers, diluents, disintegrants, binding agents, adhesives, wetting agents, polymers, lubricants, glidants, substances added to mask or counteract a disagreeable taste or odor, flavors, dyes, fragrances, and substances added to improve appearance of the composition. Acceptable carriers or excipients include citrate buffer, phosphate buffer, acetate buffer, bicarbonate buffer, stearic acid, magnesium stearate, magnesium oxide, sodium and calcium salts of phosphoric and sulfuric acids, magnesium carbonate, talc, gelatin, acacia gum, sodium alginate, pectin, dextrin, mannitol, sorbitol, lactose, sucrose, starches, gelatin, cellulosic materials (such as cellulose esters of alkanoic acids and cellulose alkyl esters), low melting wax cocoa butter, amino acids, urea, alcohols, ascorbic acid, phospholipids, proteins (for example, serum albumin), ethylenediamine tetraacetic acid (EDTA), dimethyl sulfoxide (DMSO), sodium chloride or other salts, liposomes, mannitol, sorbitol, glycerol or powder, polymers (such as polyvinylpyrrolidone, polyvinyl alcohol, and polyethylene glycols), and other pharmaceutically acceptable materials. The carrier should not destroy the pharmacological activity of the therapeutic agent and should be non-toxic when administered in doses sufficient to deliver a therapeutic amount of the agent.

The pharmaceutical composition according to the invention is preferably administered systemically by any method known in the art, including, but not limited to, intramuscular, intradermal, intravenous, subcutaneous, intraperitoneal, intranasal, oral, mucosal or external routes. The appropriate route, formulation and administration schedule can be determined by those skilled in the art. In the present invention, the pharmaceutical composition can be formulated in various ways, according to the corresponding route of administration, such as a liquid solution, a suspension, an emulsion, a syrup, a tablet, a pill, a capsule, a sustained release formulation, a powder, a granule, an ampoule, an injection, an infusion, a kit, an ointment, a lotion, a liniment, a cream or a combination thereof. If necessary, it may be sterilized or mixed with any pharmaceutically acceptable carrier or excipient, many of which are known to one of ordinary skill in the art.

The external route as used herein is also known as local administration, includes but is not limited to administration by insufflation and inhalation. Examples of various types of preparation for local administration include ointments, lotions, creams, gels, foams, preparations for delivery by transdermal patches, powders, sprays, aerosols, capsules or cartridges for use in an inhaler or insufflator or drops (e.g. eye or nose drops), solutions/suspensions for nebulisation, suppositories, pessaries, retention enemas and chewable or suckable tablets or pellets or liposome or microencapsulation preparations.

Ointments, creams and gels, may, for example, be formulated with an aqueous or oily base with the addition of suitable thickening and/or gelling agent and/or solvents. Such bases may thus, for example, include water and/or oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents.

Lotions may be formulated with an aqueous or oily base and will in general also contain one or more emulsifying agents, stabilising agents, dispersing agents, suspending agents or thickening agents.

Powders for external application may be formed with the aid of any suitable powder base, for example, talc, lactose or starch. Drops may be formulated with an aqueous or non-aqueous base also comprising one or more dispersing agents, solubilising agents, suspending agents or preservatives.

Spray compositions may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution. The aerosol composition may optionally contain additional formulation excipients well known in the art such as surfactants e.g. oleic acid or lecithin and cosolvents e.g. ethanol.

Topical preparations may be administered by one or more applications per day to the affected area; over the skin areas occlusive dressings may advantageously be used. Continuous or prolonged delivery may be achieved by an adhesive reservoir system.

The cosmetic composition according to the invention may be an aqueous phase formulation consisting essentially of water; it may also comprise a mixture of water and of water-miscible solvent (miscibility in water of greater than 50% by weight at 25°C), for instance lower monoalcohols containing from 1 to 5 carbon atoms such as ethanol or isopropanol, glycols containing from 2 to 8 carbon atoms, such as propylene glycol, ethylene glycol, 1,3-butylene glycol or dipropylene glycol, C3-C4 ketones and C2-C4 aldehydes, and glycerin. Such an aqueous formulation preferably is in a form of aqueous gel or hydrogel formulation. The hydrogel formulation comprises a thickening agent to thicken the liquid solution. Examples of the thickening agents include, but are not limited to, carbomers, cellulose base materials, gums, algin, agar, pectins, carrageenan, gelatin, mineral or modified mineral thickeners, polyethylene glycol and polyalcohols, polyacrylamide and other polymeric thickeners. The thickening agents which give the stability and optimal flow characteristics of the composition are preferably used.

The cosmetic composition according to the present invention may be in a form of emulsion or cream formulation. It can contain emulsifying surfactants. These surfactants may be chosen from anionic and nonionic surfactants. Reference may be made to the document "Encyclopedia of Chemical Technology, Kirk-Othmer", volume 22, pp. 333-432, 3rd edition, 1979, Wiley, for the definition of the properties and functions (emulsifying) of surfactants, in particular pp. 347-377 of said reference, for the anionic and nonionic surfactants.

The surfactants preferably used in the cosmetic composition according to the invention are chosen from: nonionic surfactants: fatty acids, fatty alcohols, polyethoxylated or polyglycerolated fatty alcohols such as polyethoxylated stearyl or cetylstearyl alcohol, fatty acid esters of sucrose, alkylglucose esters, in particular polyoxyethylenated fatty esters of C1-C6 alkyl glucose, and mixtures thereof; anionic surfactants: C16-C30 fatty acids neutralized with amines, aqueous ammonia or alkaline salts, and mixtures thereof. Surfactants which make it possible to obtain an oil-in-water or wax-in-water emulsion are preferably used.

The cosmetic composition according to the invention may further comprise an effective amount of a physiologically acceptable antioxidant selected from the group consisting of butylated p-cresol, butylated hydroquinone monomethyl ether, and a tocopherol.

The cosmetic composition according to the invention may further comprise natural or modified amino acid, natural or modified sterol compound, natural or modified collagen, silk protein or soy protein.

The cosmetic composition according to the invention is preferably formulated for topical application to keratin materials such as the skin, the hair, the eyelashes or the nails. They may be in any presentation form normally used for this type of application, especially in the form of an aqueous or oily solution, an oil-in-water or water-in-oil emulsion, a silicone emulsion, a microemulsion or nanoemulsion, an aqueous or oily gel or a liquid, pasty or solid anhydrous product.

The cosmetic composition according to the invention may be more or less fluid and may have the appearance of a white or colored cream, an ointment, a milk, a lotion, a serum, a paste, a mousse or a gel. It may optionally be topically applied onto the skin in the form of an aerosol, a patch or a powder. It may also be in solid form, for example, in the form of a stick. It may be used as care products and/or as makeup products for the skin. Alternatively, it may be formulated as shampoos or conditioners.

In known fashion, the cosmetic composition according to the invention may also contain additives and adjuvants that are common in cosmetics, such as hydrophilic or lipophilic gelling agents, preservatives, antioxidants, solvents, fragrances, fillers, pigments, odor absorbers and dyestuffs.

The compound can be added to a conventional food composition (i.e. the edible food or drink or precursors thereof) in the manufacturing process of the food composition. Almost all food compositions can be supplemented with the compound of the invention. The food compositions that can be supplemented with the compound of the invention include, but are not limited to, candies, baked goods, ice creams, dairy products, sweet and flavor snacks, snack bars, meal replacement products, fast foods, soups, pastas, noodles, canned foods, frozen foods, dried foods, refrigerated foods, oils and fats, baby foods, or soft foods painted on breads, or mixtures thereof.

While not wishing to be limited by theory, it is Applicant's belief that the method according to the invention is treating the brain cancer through inducing reactive oxygen species (ROS) in a cancer cell or inducing lipid oxidation on a cell membrane and membranes of inner cell organelles in a cancer cell.

The reduction-oxidation (redox) state is used to describe the balance of GSH/GSSG, NAD⁺/NADH and NADP⁺/NADPH in a biological system such as a cell or organ. The redox state is reflected in the balance of several sets of metabolites, whose interconversion is dependent on these ratios. An abnormal redox state can develop in a variety of deleterious situations. Free radical reactions are redox reactions that occur as a part of homeostasis and killing microorganisms, where an electron detaches from a molecule and then reattaches almost instantaneously. Reactive oxygen species (ROS) are a part of redox molecules and can become harmful to the human body if they do not reattach to the redox molecule or an antioxidant. Unsatisfied free radicals can spur the mutation of cells they encounter and are, thus, causes of cancers and other diseases (R Kohen, A Nyska, Oxidation of Biological Systems: Oxidative Stress Phenomena, Antioxidants, Redox Reactions, and Methods for Their Quantification. Toxicologic pathology, 30(6): 620-650, 2002; Trachootham D, Alexandre J, Huang P. Targeting cancer cells by ROS-mediated mechanisms: a radical therapeutic approach? Nat Rev Drug Discov. Jul;8(7):579-91. 2009).

The compound with (substituted phenyl)-propenal moiety regulates the homeostasis of redox condition in various kind of cells, tissues and organs. In tumor, the compound with (substituted phenyl)-propenal moiety induces lipid oxidation on the cell membrane and membranes of inner cell organelles and leads to high level of the oxidative stress that contributes to cell damages and cell death. Sequentially, the lipid ROS generated by the compounds with (substituted phenyl)-propenal moiety causes multiple cellular and molecular responses to echo the cell damages such as increase of chaperone expression, activation of ubiquitin-proteasomal system, change of mitochondrial biogenesis, unfolded protein response (UPR), disruption of proteostasis, autophagy, and apoptosis. These multiple cellular reactions finally lead the cancer cells to die through apoptosis and autophagy.

While not wishing to be limited by theory, it is Applicant's belief that the method according to the invention is treating the brain cancer through increasing chaperone expression.

While not wishing to be limited by theory, it is Applicant's belief that the method according to the invention is treating the brain cancer through activating ubiquitin-proteasomal system.

While not wishing to be limited by theory, it is Applicant's belief that the method according to the invention is treating the brain cancer through changing mitochondrial biogenesis.

While not wishing to be limited by theory, it is Applicant's belief that the method according to the invention is treating the brain cancer through activating unfolded protein response (UPR).

While not wishing to be limited by theory, it is Applicant's belief that the method according to the invention is treating the brain cancer through disrupting proteostasis.

While not wishing to be limited by theory, it is Applicant's belief that the method according to the invention is treating the brain cancer through activating autophagy.

While not wishing to be limited by theory, it is Applicant's belief that the method according to the invention is treating the brain cancer through activating apoptosis.

In one preferred embodiment of the invention, the brain cancer is a cancer with multidrug resistance; preferably, the brain cancer is a temozolomide -resistant brain cancer.

Preferably, the brain cancer is a primary brain cancer. The primary brain cancer is preferably gliomas, meningiomas, pituitary adenomas, vestibular schwannomas, primitive neuroectodermal tumors (medulloblastomas), germ cell tumors, neuroblastoma or craniopharyngiomas. Preferably, the gliomas are glioblastomas, glioblastoma multiforme, astrocytomas, oligodendrogliomas, or ependymomas.

Using a variety of cellular models of GMB, the Applicant obtained vast experimental evidence that ASC-JM17 exhibits toxicity in glioblastoma cells by inducing oxidative stress and by activating both extrinsic and intrinsic apoptotic pathways. Further, in the validated animal model of GBM, ASC-JM17 demonstrated the ability to alleviate clinically meaningful morbidity (weight loss) and, most importantly, was able to improve the overall survival of experimental animals. The fact that anti-cancer effects of ASC-JM17 were demonstrated in the models of the TMZ sensitive and TMZ-resistant GBM makes these finding specifically relevant to the unmet medical need of the GBM patients.

Taken together, these data strongly support that ASC-JM17 may provide benefit to the GMB patients.

Preferably, the brain cancer is a secondary brain cancer. In one preferred embodiment of the invention, a primary site of the secondary brain cancer is breast cancer, colorectal cancer, kidney cancer, lung cancer, lymphoma, myeloma, pancreatic cancer, liver cancer, prostate cancer, bladder cancer, skin cancer, non-Hodgkin lymphoma, leukemia, uterine cancer, adrenal cancer, stomach cancer, bile duct cancer, bone cancer, ovarian cancer, throat cancer, oral cancer, head and neck cancer, thyroid cancer, soft tissue sarcoma, testicular cancer, vaginal cancer or melanoma.

The following examples are provided to aid those skilled in the art in practicing the present invention.

### EXAMPLES

### Example 1: ASC-JM17 (JM17) efficacy in In Vitro models of glioblastoma multiforme (GBM)

### JM17 Selectively Exhibits Cytotoxic Effects on TMZ-resistant Glioblastoma

The effects of JM17 on proliferation of GBM cells was studied in several cell lines independently derived from the GBM cancers. First, well characterized and commercially available A172 and U87MG cells were purchased from the ATCC (ATCC® CELL LINES BY GENE MUTATION. 2019). Second, the primary glioblastoma cells, Pt#3, were derived from a GBM patient by following a published procedure (Chang KY, Hsu TI, Hsu CC, et al. Specificity protein 1-modulated superoxide dismutase 2 enhances temozolomide resistance in glioblastoma, which is independent of O(6)-methylguanine-DNA methyltransferase. Redox Biol. 2017;13:655-664; Chuang JY, Lo WL, Ko CY, et al. Upregulation of CYP17A1 by Sp1-mediated DNA demethylation confers temozolomide resistance through DHEA-mediated protection in glioma. Oncogenesis. 2017;6(5):e339). Additionally, temozolomide (TMZ)-resistant clones of the U87MG and A172 were created following published protocols (Chang KY, Hsu TI, Hsu CC, et al. Specificity protein 1-modulated superoxide dismutase 2 enhances temozolomide resistance in glioblastoma, which is independent of O(6)-methylguanine-DNA methyltransferase. Redox Biol. 2017;13:655-664; Chuang JY, Lo WL, Ko CY, et al. Upregulation of CYP17A1 by Sp1-mediated DNA demethylation confers temozolomide resistance through DHEA-mediated protection in glioma. Oncogenesis. 2017;6(5):e339). The cellular viability assay was performed as follows. Cells, grown to the sub confluency, were maintained in the presence of the vehicle (DMSO) or 1 µM, 2 µM, 5 µM and 10 µM JM17 for 24 h, 48 h or 72 h (FIG. 1). Then cells were harvested, and cellular viability was measured by MTT Cell Proliferation Assay per the manufacturer's instructions (Chang KY, Hsu TI, Hsu CC, et al. Specificity protein 1-modulated superoxide dismutase 2 enhances temozolomide resistance in glioblastoma, which is independent of O(6)-methylguanine-DNA methyltransferase. Redox Biol. 2017;13:655-664).

As shown in FIGs. 1A and 1B, JM17 significantly decreased proliferation of TMZ-sensitive cells A172 and Pt#3 as well as the TMZ-resistant glioblastoma cell line, A172-R. Moreover, proliferation of both TMZ-sensitive U87MG and TMZ-resistant U87MG-R was inhibited by JM17 in time- and dose-dependent manners (FIG. 1B).

Of note, JM17 at the concentrations up to 10 µM had no effect on the survival of normal mouse astrocytes (data not shown), indicating low toxicity of the drug towards uncancerous cells.

To further elucidate the mechanism of the JM17 action on the glioblastoma cells and to assess whether JM17 induces apoptosis, the activities of caspases 3/7, 8 and 9 were measured in the supernatants of cells treated with the drug. It was found that JM17 at 2 µM significantly increased the activities of caspases 3/7, 8 and 9 in U87MG and Pt#3 cells (FIG. 2). These data strongly support the notion that JM17 exhibits toxicity in glioblastoma cells by activating both extrinsic and intrinsic apoptotic pathways.

### JM17 Induces Oxidative Stress in the Glioblastoma Cells by Inhibiting GSHR Activity

Cellular redox imbalance has been found in GBM, which indicates that pharmaceutical interventions improving cellular redox homeostasis may provide a positive therapeutic impact (Gorrini C, Harris IS, Mak TW. Modulation of oxidative stress as an anticancer strategy. Nat Rev Drug Discov. 2013;12(12):931-947; Salazar-Ramiro A, Ramirez-Ortega D, Perez de la Cruz V, et al. Role of Redox Status in Development of Glioblastoma. Front Immunol. 2016;7:156). Since the increase in the ROS levels induces apoptosis, several compounds that increase ROS production have been considered as therapeutic agents against GBM (Salazar-Ramiro A, Ramirez-Ortega D, Perez de la Cruz V, et al. Role of Redox Status in Development of Glioblastoma. Front Immunol. 2016;7:156).

As shown in FIGs. 3A to 3D, JM17 at 2 µM significantly increased ROS production in U87MG cells. Further, JM17 significantly increased H₂O₂ level concurrently with the reduction in the GSH/GSSG ratio (FIGs. 3B to 3C), indicating that JM17 is a strong inducer of oxidative stress in glioblastoma. Furthermore, glutathione reductase (GSHR) activity, which is responsible for maintaining the level of reduced GSH, was significantly decreased by JM17 in a dose-dependent manner (FIG. 3D). Taken together, these data support the hypothesis that the glioblastoma-suppressive effect of JM17 is mediated by ROS accumulation in the cancer cells.

### Example 2: ASC-JM17 (JM17) efficacy in the glioblastoma multiforme (GBM) animal model

### JM17 inhibits the growth of glioblastoma in orthotopic mice model

To further investigate tumor-suppressive properties of JM17 *in vivo*, a mouse model of GBM with intracranially transplanted luciferase-expressed U87MG cells was utilized.

The experiment was performed as follows. NOD-SCID male mice (8-week-old) were purchased from BioLASCO® Taiwan Co., Ltd. (Taipei, Taiwan), and maintained at the National Health Research Institutes (Tainan, Taiwan). For intracranial transplantation, luciferase-expressed U87MG cells (2 x 10⁵) were injected into the cortex at the depth of 3 mm using stereotactic guidance and microprocessor single syringe (Harvard Apparatus®, Holliston, MA, USA). After 10 days of transplantation, JM17 was intravenously administrated three times per week. To prepare the formulation for intravenous injection, JM17 stock in DMSO (50 mg/ml) was diluted in the mixture of PBS and Tween 80 (MilliporeSigma® Corporate). Control mice were treated with the vehicle (DMSO/PBS/Tween). On the Day 10 post transplantation, the mice were randomized into four treatment groups (five mice per group) and treated intravenously with the vehicle or JM17 at the dose of 20 mg/kg, 40 mg/kg and 80 mg/kg three times per week. The treatment was stopped at the Day 63. Luciferase activity was monitored by IVIS 200 system (Xenogen® Corporation, Alameda, CA, USA) as a measure of the tumor size.

The results of this experiment are presented in FIGs. 4A to 4C. As evident from FIG. 4A, the luciferase activity of U87MG cells was readily detectable at day10, indicating that the tumor was established by the time the treatment was initiated. As expected, the tumor was rapidly growing in the control group receiving vehicle (DMSO). The growth of transplanted U87MG cells was significantly inhibited in mice injected with JM17 (FIGs. 4A to 4B). Importantly, this inhibition was concentration depended (FIG. 4A).

Further, the overall survival of mice transplanted with luciferase-expressed U87MG cells was affected by the JM17 treatment. All the animals from the control group did not survive post day 34 (these mice died on days 27-34). The survival period was significantly extended to Days 34-41, 38-66 and 44-76 by 20, 40 and 80 mg/kg of JM17 administration, respectively (FIG. 4C).

These data provide evidence in the support of the hypothesis that JM17 may suppress tumor growth and increase overall survival in GBM patients.

### JM17 inhibits the growth of TMZ-resistant glioblastoma in orthotopic mice model

Having found that JM17 inhibits proliferation of the TMZ-resistant cells U87MG-R in culture, the tumor-suppressive effects of JM17 were further evaluated in an intracranial brain tumor mouse model developed by Chang et al (Chang KY, Hsu TI, Hsu CC, et al. Specificity protein 1-modulated superoxide dismutase 2 enhances temozolomide resistance in glioblastoma, which is independent of O(6)-methylguanine-DNA methyltransferase. Redox Biol. 2017;13:655-664). Briefly, a skull burr hole was first created in the right frontal brain area. An ultra-fine needle was then inserted to a depth of 3 mm using stereostatic guiding, and 5 × 10⁵ TMZ-resistant cells were injected.

Twenty-eight-week-old nude mice received U87MG-R transplantation. Five days later, these animals were randomized into four treatment groups (7 animals per group), which received TMZ (15 mg/kg) by oral gavage and JM17 by the intravenous injection three times per week (Day 1, 3, 5 of the week) as follows: Control (untreated), TMZ plus vehicle, TMZ plus JM17 (5 mg/kg) and TMZ plus JM17 (20 mg/kg) (FIG. 5).

The median survival was 21 days in the control (untreated) group (FIG. 6). As expected, TMZ alone (TMZ plus vehicle) did not influence the survival of animals bearing TMZ-resistant tumors. The median survival was 20 days in the TMZ plus vehicle group. The median survival of mice was increased by JM17 treatment to 24 days (at 5 mg/kg) and to 28 days (at 20 mg/kg). The statistical analysis by Log-Rank test showed that the extension of the survival period from 20 to 28 days was statistically significant (p<0.01).

The assessment of body weight is a validated approach used to monitor morbidity in the experimental mouse cancer studies as a clinically relevant endpoint (Paster EV, Villines KA, Hickman DL. Endpoints for mouse abdominal tumor models: refinement of current criteria. Comp Med. 2009;59(3):234-241). As shown in FIG. 7, GBM-inoculated mice keep their weight for about 4 to 6 days and then start losing weight, which is indicative of the progression of the disease. Since the GBM in these animals is TMZ-resistant, it is not surprising that TMZ treatment does not prevent weight loss. In contrast, JM17 treatment significantly reduced body mass wasting at both dosage levels used in these experiments when compared to the TMZ treated control (FIG. 7).

While the present invention has been described in conjunction with the specific embodiments set forth above, many alternatives thereto and modifications and variations thereof will be apparent to those of ordinary skill in the art. All such alternatives, modifications and variations are regarded as falling within the scope of the present invention.

## Claims

1. A pharmaceutical composition for use in a method for treating a brain cancer in a subject in need of such treatment, comprising administrating to said subject the pharmaceutical composition comprising an effective amount of a compound according to formula VIII and optionally a pharmaceutically acceptable carrier or excipient; 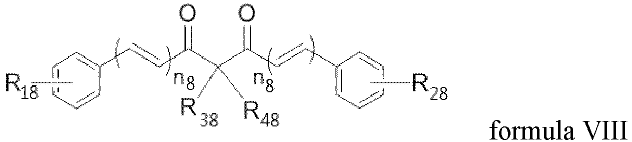
R₁₈ and R₂₈ are mono- or di-substituted groups and independently selected from the group consisting of a methoxy group, a hydroxyl group, and an alkyl sulfonyl group; R₃₈ is selected from the group consisting of 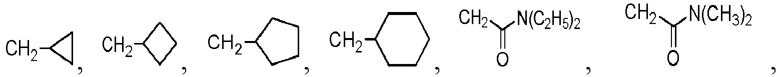 (CH₂)₃CH₃, 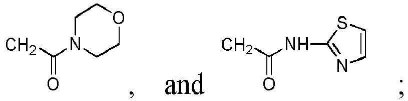 and
R₄₈ is selected from the group consisting of CH₃, H, F and Cl; and
n₈ is 1 or 2.

2. The pharmaceutical composition for use in a method for treating a brain cancer according to claim 1, wherein R₁₈ and R₂₈ are di-substituted methoxy groups, R₃₈ is 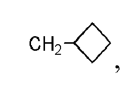 ₄₈ is H, and n₈ is 1.

3. The pharmaceutical composition for use in a method for treating a brain cancer according to claim 1 or 2, wherein the cancer is a cancer with multidrug resistance.

4. The pharmaceutical composition for use in a method for treating a brain cancer according to at least one of the preceding claims, wherein the brain cancer is a temozolomide -resistant brain cancer.

5. The pharmaceutical composition for use in a method for treating a brain cancer according to at least one of the preceding claims, wherein the brain cancer is a primary brain cancer.

6. The pharmaceutical composition for use in a method for treating a brain cancer according to claim 5, wherein the primary brain cancer is gliomas, meningiomas, pituitary adenomas, vestibular schwannomas, primitive neuroectodermal tumors (medulloblastomas), germ cell tumors, neuroblastoma or craniopharyngiomas.

7. The pharmaceutical composition for use in a method for treating a brain cancer according to claim 6, wherein the gliomas are glioblastomas, glioblastoma multiforme, astrocytomas, oligodendrogliomas, or ependymomas.

8. The pharmaceutical composition for use in a method for treating a brain cancer according to at least one of claims 1 to 4, wherein the cancer is a secondary brain cancer.

9. The pharmaceutical composition for use in a method for treating a brain cancer according to claim 8, wherein a primary site of the secondary brain cancer is breast cancer, colorectal cancer, kidney cancer, lung cancer, lymphoma, myeloma, pancreatic cancer, liver cancer, prostate cancer, bladder cancer, skin cancer, non-Hodgkin lymphoma, leukemia, uterine cancer, adrenal cancer, stomach cancer, bile duct cancer, bone cancer, ovarian cancer, throat cancer, oral cancer, head and neck cancer, thyroid cancer, soft tissue sarcoma, testicular cancer, vaginal cancer or melanoma.
